# EUROPÄISCHE PATENTSCHRIFT

(11) **EP 0 697 833 B1**
(45) Veröffentlichungstag und Bekanntmachung des Hinweises auf die Patenterteilung: **26.01.2000**
(21) Anmeldenummer: 95913079.0
(22) Anmeldetag: 09.03.1995
(51) Int. Cl.: A61B 1/00

(54) **ENDOSKOP MIT SEITLICHEN KABELABGÄNGEN**
ENDOSCOPE WITH LATERAL CABLE OUTLETS
ENDOSCOPE A DEPARTS LATERAUX DE CABLES

(30) Priorität: 12.03.1994 DE 4408393
(43) Veröffentlichungstag der Anmeldung: 28.02.1996
(73) Patentinhaber: OLYMPUS WINTER & IBE GmbH, 22045 Hamburg (DE)
(72) Erfinder: KRAAS, Mathias, D-22880 Wedel (DE); PROSCHWITZ, Detlef, D-22927 Grosshansdorf (DE); WOSNITZA, Thomas, D-21337 Lüneburg (DE); WIEGAND, Michael, D-21509 Glinde (DE)
(74) Vertreter: Schaefer, Konrad, Dipl.-Phys.
(86) Internationale Anmeldenummer: EP9500887
(87) Internationale Veröffentlichungsnummer: WO9524856

(56) Entgegenhaltungen:
- WO-A-93/25137
- DE-U- 8 505 354
- FR-A- 1 231 169

## Beschreibung

Die Erfindung betrifft ein Endoskop der im Oberbegriff des Anspruchs 1 genannten Art.

Ein gattungsgemäßes Gerät ergibt sich aus der
DE 41 05 326 C2.

In dieser Schrift wird ein urologischer Resektor dargestellt, der im folgenden als Resektor bezeichnet wird.

Bei bekannten Instrumenten sind seitliche Kabelabgänge vorgesehen. Im Falle des Resektors sind dies ein Lichtleiterkabel und ein HF-Kabel.

Der Resektor weist ein Arbeitsinstrument auf mit dem unter optischer Beobachtung durch die Optik in einem Operationsfeld vor dem distalen Ende des Schaftteiles gearbeitet werden kann. Dabei handelt es sich um eine Schneidschlinge, die von der Griffeinrichtung her zu axialer Bewegung betätigbar ist und die an eine HF-Leitung angeschlossen ist, welche ebenfalls einen seitlichen Kabelabgang aufweist.

Bei Arbeiten im Operationsgebiet muß das Arbeitsinstrument an die Operationsstelle herangeführt werden. Dazu ist häufig auch eine Verdrehung des Arbeitsinstrumentes erforderlich. Dies wird besonders deutlich im Falle des Resektors, mit dem in der Prostata im vollen 360°-Winkelbereich ständig hin- und hergedreht werden muß.

Die Steuerung der Drehung des Arbeitsinstrumentes erfolgt dabei stets über die Griffeinrichtung, die von der Hand des Operateurs gehalten und unter Drehmitnahme des Arbeitsinstrumentes gedreht wird.

Dabei bilden bei solchen Drehbewegungen die seitlichen Kabelabgänge ein großes Hindernis. Da sie nach dem Stand der Technik mit der Griffeinrichtung drehfest verbunden sind, werden bei jeder Drehbewegung die Kabel mitgeschwenkt. Bei mehrfacher Drehung über größere Winkel wickeln sich die Kabel um den Endkörper. Außerdem stört das hohe Gewicht der seitlich abgehenden Kabel, insbesondere des sehr schweren Lichtleiterkabels, wodurch sich in unterschiedlicher Winkelstellung unterschiedliche und somit stark störende Drehmomente ergeben.

Bei dem bekannten Resektor ist die Kamera am proximalen Ende des Endoskopes an das nach dem Stand der Technik vorgesehene Okular angesetzt, wobei das Kabel der Kamera etwa in Richtung der Schaftachse von der Kamera abgehend verläuft. Dieses Kabel übt zwar keine störenden Drehmomente aus, behindert aber den Operateur bei der Bedienung des Endoskopes, da es sich beim Drehen der Griffeinrichtung um seinen Unterarm wickelt.

Ferner ungünstig bei dem bekannten Resektor ist die Lage des als Daumenring ausgebildeten proximalen Griffteiles, das seitlich neben dem Endkörper angeordnet ist. Bei Drehungen muß daher stets mit dem Daumen um den Endkörper herumgegriffen werden, wodurch die freie Drehhandhabung eingeschränkt wird.

Weiterhin bekannt sind aus der
DE 39 28 421 A1

Pistolengriffinstrumente mit im Griff angeordneten Kabelabgängen für das Kamerakabel und das Lichtleiterkabel. Pistolengriffinstrumente haben aber den Nachteil, daß bei Ihnen Drehungen nur schwer möglich sind, da durch die Fixierung der Hand am Pistolengriff nur geringfügige Drehungen des Instrumentes möglich sind. Eine Drehsteuerung des Arbeitsinstrumentes über die Griffeinrichtung ist bei solchen Instrumenten daher nicht möglich. Dazu sind gesonderte Dreheinrichtungen erforderlich, wie sich aus der umfangreichen Literatur zu Pistolengriffinstrumenten ergibt.

Die Aufgabe der vorliegenden Erfindung besteht darin, ein Endoskop der eingangs genannten Art zu schaffen, das eine leichte Drehsteuerung durch den Operateur ohne Behinderung durch Kabel ermöglicht.

Diese Aufgabe wird erfindungsgemäß mit den Merkmalen des Kennzeichnungsteiles des Anspruchs 1 gelöst.

Bei dieser Konstruktion sind die Kabelabgänge, wie bei den Geräten nach dem Stand der Technik, seitlich vom Endkörper abgehend ausgebildet, jedoch an einem Gehäuse, das gegenüber dem Arbeitsinstrument und somit auch gegenüber der Griffeinrichtung frei drehbar ist. Die Kabel können also unter ihrem Eigengewicht nach unten hängen, wobei die Kabelabgänge nach unten weisen. Es ist dabei eine freie Drehsteuerung des Arbeitsinstrumentes durch die Griffeinrichtung möglich, ohne daß die Kabel störend mitdrehen. Da die Kabelabgänge distal vom proximalen Griffteil liegen, ist dieses durch den Daumen des Operateurs allseitig völlig frei bedienbar, wodurch die freie Drehsteuerung gefördert wird. Das Arbeiten mit einem solchen Endoskop unter Ausführung von Drehbewegungen, insbesondere im Falle eines urologischen Resektoskopes, bei dem solche Drehbewegungen sehr häufig sind, wird also wesentlich erleichtert.

Auch bei anderen Anwendungen, beispielsweise in der Gynäkologie ergeben sich mit dem erfindungsgemäßen Instrument wesentliche Bedienungsvorteile.

Vorteilhaft sind die Merkmale des Anspruches 2 vorgesehen. Ein solches Endoskop ist konstruktiv sehr einfach zu realisieren. Der zur Drehsteuerung dienende distale Griffteil ist distal vom drehbaren Gehäuse angeordnet, so daß er unmittelbar zur Drehsteuerung in herkömmlicher Weise dienen kann ohne Beeinflussung der freien Drehbarkeit des Gehäuses, dessen Drehlagerung sehr einfach ausgeführt werden kann.

Alternativ sind die Merkmale des Anspruches 3 vorgesehen. Bei dieser Ausführung ist die gesamte Griffeinrichtung proximal von dem drehbaren Gehäuse vorgesehen, so daß sie insgesamt, also mit allen Fingern der bedienenden Hand des Operateurs völlig frei von Kabeln von allen Seiten her gleich gut bedienbar ist. Die Übertragung der Drehsteuerung und gegebenenfalls der Axialsteuerung des Arbeitsinstrumentes um das frei drehbare Gehäuse herum oder durch dieses hindurch bedingt bei dieser Konstruktionsweise allerdings einen erhöhten konstruktiven Aufwand.

Mehrere Kabelabgänge können jeweils an eigenen, getrennt voneinander drehbar gelagerten Gehäusen vorgesehen sein. Vorteilhaft sind jedoch die Merkmale des Anspruches 4 vorgesehen, durch die die Konstruktion vereinfacht wird. Außerdem addiert sich das Gewicht der herabhängenden mehreren Kabel, so daß auch bei Reibung in der Drehlagerung des Gehäuses ein sicheres Herabhängen der Kabel in stets derselben Winkelstellung, in der sie nicht stören, gewährleistet bleibt.

Vorteilhaft sind die Merkmale des Anspruches 5 vorgesehen. Auf diese Weise wird auch das Kabel einer Kamera weniger störend verlegt als bei den eingangs diskutierten Konstruktionen nach dem Stande der Technik. Dabei ist die Kamera distal vom proximalen Griffteil angeordnet, wodurch diese in ihrer Konstruktionsweise freier gewählt werden kann und keine Rücksicht auf eine axial anzuordnende Kamera nehmen muß, wie dies beispielsweise im Falle der DE 41 05 326 C2 der Fall ist.

Dabei sind vorteilhaft die Merkmale des Anspruches 6 vorgesehen. Wie bereits aus der DE 41 05 326 C2 bekannt, wird auf diese Weise erreicht, daß die Kamera bei Drehbewegungen winkelfest bleiben kann, wobei die von dem Gehäuse herabhängenden Kabel bei Drehbewegungen an der Griffeinrichtung die Kamera festhalten. Das bei Drehbewegungen stillstehende Bild der Kamera erleichtert dem Operateur den Überblick. Dabei sorgt die Anordnung der Optikachse in der Lagerachse des Gehäuses dafür, daß bei Drehen der Optik um die Optikachse das Bild der Kamera stehen bleibt und nicht "eiert".

Vorteilhaft sind die Merkmale des Anspruches 7 vorgesehen. Auf diese Weise kann in vorteilhafter Bauausführung die Optik zur Halterung des Arbeitsinstrumentes oder als Teil von diesem verwendet werden. Genausogut kann aber auch die Optik drehfest mit dem Gehäuse verbunden werden. Dies kann beispielsweise von Vorteil sein, wenn aus bestimmten Gründen das Mitdrehen der Optik und des Objektives bei Drehverstellung des Arbeitsinstrumentes verhindert werden soll.

Ist eine Kamera vorgesehen, die zur Vereinfachung des Kabelanschlusses und zur Drehfesthaltung des Bildes mit dem Gehäuse verbunden ist und die das in der Lagerachse des Gehäuses liegende proximale Ende der Optik beobachtet, so muß die Kamera unmittelbar in der Lagerachse angeordnet sein oder wenigstens mit einer Strahlumlenkeinrichtung in diese eingreifen. Bei einem Endoskop nach Anspruch 2 ergeben sich hieraus keine Probleme, wohl aber bei einem Endoskop nach Anspruch 3, bei dem die Drehsteuerung von dem distalen Griffteil über die Stelle hinweg erfolgen muß, an der die Kamera oder die Strahlumlenkeinrichtung in die Drehachse eingreift. Da dabei mindestens für die Drehübertragung der Griffeinrichtung auf das Arbeitsinstrument eine Verbindung zwischen diesen Teilen bestehen muß, ergibt sich eine Drehwinkelbeschränkung für das Gehäuse und die Kamera gegenüber dem Arbeitsinstrument, da die Kamera nicht an der Drehverbindung vorbeigeschwenkt werden kann. Zur Lösung dieses Problemes sind vorteilhaft die Merkmale des Anspruches 8 vorgesehen. Nach dieser Konstruktion sind das Arbeitsinstrument und die Griffeinrichtung innerhalb des Gehäuses mit einem Zahnradgetriebe gekoppelt, das nach Art eines Planetengetriebes ausgebildet ist und bei Drehfesthaltung des Gehäuses eine Drehkoppelung des Arbeitsinstrumentes an die Griffeinrichtung über das Zahnradgetriebe gewährleistet. Der Vorteil dieser Konstruktion besteht darin, daß die Koppelung des proximalen Endes des Arbeitsinstrumentes und des distalen Endes der Griffeinrichtung über eine außerhalb der Lagerachse des Gehäuses angeordnete Verbindungswelle erfolgt. Im axialen Bereich der Verbindungswelle ist daher das Gebiet der Lagerachse frei zur Aufnahme der Kamera. Da die Verbindungswelle mit dem Gehäuse und somit mit der Kamera dreht, kann die Kamera gegenüber der Griffeinrichtung uni beliebige Winkel, also über 360° hinaus verdreht werden. Das Zahnradgetriebe bietet außerdem den Vorteil, daß die Drehkoppelung des Arbeitsinstrumentes an die Griffeinrichtung mit unterschiedlicher Übersetzung gewählt werden kann, entweder 1 : 1, also wie bei starrer Verbindung, oder auch mit Übersetzung ins Schnelle oder ins Langsame. Es kann die Übersetzung des Zahnradgetriebes also beispielsweise so gewählt werden, daß schon bei geringer Verdrehung der Griffeinrichtung eine Verdrehung des Arbeitsinstrumentes über volle 360° erreicht wird. Andererseits kann auch zur besonders feinfühligen Verdrehung des Arbeitsinstrumentes die Übersetzung so gewählt werden, daß für eine bestimmte Verdrehung des Arbeitsinstrumentes eine größere Winkelbewegung der Griffeinrichtung erforderlich ist. Die Übersetzung kann mit einem Schaltgetriebe verstellbar sein.

Aufgrund anderer technischer Probleme, beispielsweise beim Anschluß der Kabel innerhalb des Gehäuses, kann es erforderlich sein, den Drehwinkel des Gehäuses gegenüber dem Arbeitsinstrument zu begrenzen, beispielsweise so, daß zwar der volle 360°-Winkelbereich erreicht, aber nicht wesentlich überschritten wird. Dazu sind vorteilhaft die Merkmale des Anspruches 9 vorgesehen. Hierbei kann auf konstruktiv besonders einfache Weise durch Ergänzung des Zahnradgetriebes um nur ein mitdrehendes Zahnrad dieses zur Drehbewegung zwischen Anschlägen verwendet werden. Die Übersetzung zwischen diesem mitlaufenden Zahnrad und dem Arbeitsinstrument kann beliebig gewählt werden, so daß sich beispielsweise aus einer geringen Drehbewegung des mitlaufenden Zahnrades zwischen den Endanschlägen eine gewünschte Drehbewegung des Arbeitsinstrumentes über einen Bereich größer als 360° ergibt.

Arbeitsinstrumente müssen in der Regel in irgendeiner Weise betätigt werden. Üblicherweise erfolgt dieses durch eine in der Schafteinrichtung verlegte Betätigungsstange, mit der beispielsweise im Falle eines urologischen Resektors die HF-beaufschlagte Schneidschlinge zur Schneidbewegung - je nach Ausführung der Griffeinrichtung - vor- oder zurückgeschoben wird. Im Falle einer Zange werden deren Backen über die Betätigungsstange geöffnet und geschlossen. Die Betätigung erfolgt in üblicher Weise voll der Griffeinrichtung her. Vorteilhaft sind daher die Merkmale des Anspruches 10 vorgesehen. Die Schiebestange der Betätigungseinrichtung ist im Gehäuse gelagen. Dadurch steht sie stets in einer Winkelstellung, in der sie weder die Kamera noch die Kabel behindern kann. An ihren Enden steht sie in längsfestem Dreheingriff mit koaxialen Flächen am Arbeitsinstrument und an der Griffeinrichtung und kann zwischen diesen eine Längsverstellbewegung zur Betätigung des Arbeitsinstrumentes übertragen.

Bei Verwendung einer Griffeinrichtung mit Daumenring, wie sie aus der DE 41 05 326 C2 bekannt ist, sind vorteilhaft die Merkmale des Anspruches 11 vorgesehen. Aus ergonomischen Gründen ist die Anordnung des Daumenringes in der Lagerachse, also in der Drehachse des Arbeitsinstrumentes, vorzuziehen, da sie dann eine besonders feinfühlige Drehverstellung der Griffeinrichtung durch die Hand des Operateurs erlaubt. Diese besonders günstige Anordnung der Griffeinrichtung zu ermöglichen, stellt einen wesentlichen Vorteil der vorliegenden Erfindung dar. Das Fingergriffstück, an dem die dem Daumen gegenüberliegenden Finger der das Endoskop betätigenden Hand angreifen, kann, wie bei dem genannten Stand der Technik als Griffstange ausgebildet sein, kann aber auch als rotationssymmetrische Scheibe oder insbesondere als Ring ausgebildet sein, an dem nur die Fingerspitzen angreifen. Dies erlaubt in besonders vorteilhafter Weise die Ausnutzung der Vorteile der vorliegenden Erfindung, nämlich eine in allen Winkelstellungen der Griffeinrichtung gleich gute Angriffsmöglichkeit für die Hand des Operateurs, also ein sehr gutes Arbeiten in unterschiedlichen Winkelstellungen des Arbeitsinstrumentes. Dabei kann auch an Stelle des Daumenringes herkömmlicher Ausbildung eine rotationssymmetrische Scheibe oder ein Ring vorgesehen sein, was unter Umständen und je nach ergonomischen Bedürfnissen des Operateurs Bedienungsvorteile bringen kann.

Die von den Kabelabgängen nach innen angeschlossenen Kabel verlaufen meist zu Einrichtungen, die mit dem Arbeitsinstrument drehen. Es handelt sich beispielsweise um elektrische Anschlüsse zum Arbeitsinstrument oder um das Lichtleiterkabel, das in der Regel innerhalb der Optik verläuft, die zumeist mit dem Arbeitsinstrument drehfest verbunden ist. Dazu sind vorteilhaft die Merkmale des Anspruches 12 vorgesehen. Die Drehverbindungen können auf unterschiedliche Weise vorgesehen sein, beispielsweise über elektrische oder optische Schleifringverbindungen.

Ein Lichtleiter ist, wenn die Optik drehfest mit dem Arbeitsinstrument verbunden ist, vorteilhaft über eine Drehverbindung mit den Merkmalen des Anspruches 13 angeschlossen. Solche Drehverbindungen haben sich im Stand der Technik bewährt und zeichnen sich durch große Einfachheit aus.

Eine HF-Leitung, die z.B. bei urologischen Resektoskopen mit HF-beaufschlagter Schneidschlinge erforderlich ist, ist vorteilhaft mit einer Drehverbindung nach den Merkmalen des Anspruches 14 angeschlossen. Diese Drehverbindung vermeidet die sonst zur Drehverbindung von HF-Leitungen vorgesehenen Schleifringe und zeichnet sich durch hohe Langzeitfestigkeit und Isoliersicherheit aus.

Bei Verwendung einer Kamera ist diese vorteilhaft gemäß den Merkmalen des Anspruches 15 angeordnet. Die Kamera selbst sitzt hierbei in der Seitenwand des Gehäuses und kann dort sehr einfach abnehmbar ausgebildet sein, beispielsweise zu Reinigungszwecken des Endoskopes.

Aus der DE 41 01 472 A1 ist ein urologisches Resektoskop bekannt, bei dem ein Außenschaft frei drehbar auf der Schafteinrichtung gelagert ist. Diese Konstruktion hat den Vorteil, daß der Außenschaft bei Verdrehungen des Arbeitsinstrumentes ohne Reibbeeinträchtigung des Patienten drehtest liegen bleibt. Bei Verwendung eines solchen Endoskopes sind vorteilhaft die Merkmale des Anspruches 16 vorgesehen. Auf diese Weise wird das Gehäuse mit den Kabelabgängen und gegebenenfalls mit der Kamera durch den im Patienten drehtest liegenden Außenschaft drehtest gehalten. Bei dieser Konstruktion ergibt sich die Möglichkeit, sämtliche vom Endoskop abgehenden Anschlüsse, also sowohl die elektrischen und optischen Kabel als auch die zur Zu- und Abfuhr der Spülflüssigkeit erforderlichen Schläuche bei den erforderlichen Drehbewegungen des Arbeitsinstrumentes drehtest zu lagern. Ein wesentlicher Vorteil bei einer solchen Konstruktion ist es auch, daß die Drehsteuerung des Arbeitsinstrumentes wesentlich leichtgängiger erfolgt, da die Reibung des Schaftes im Patienten ausgeschaltet ist.

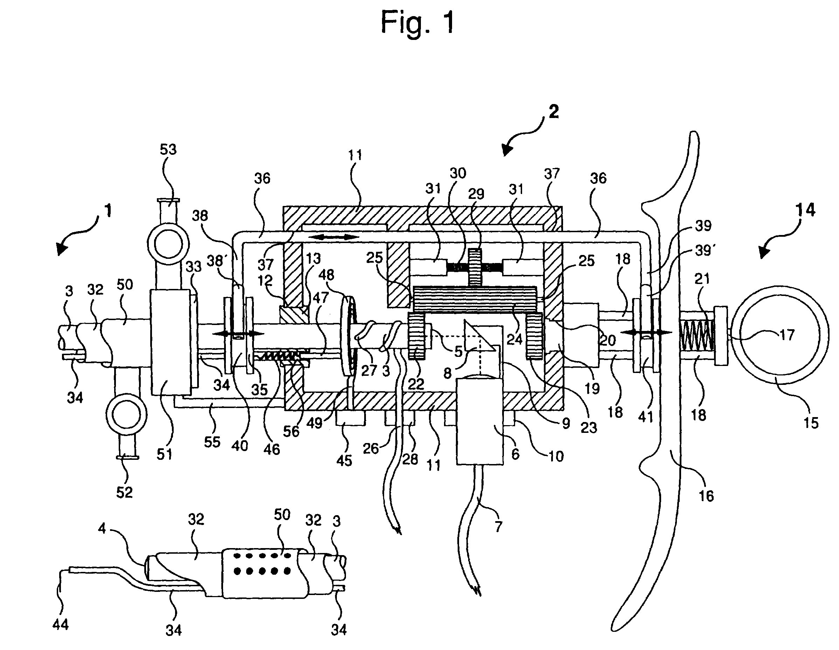

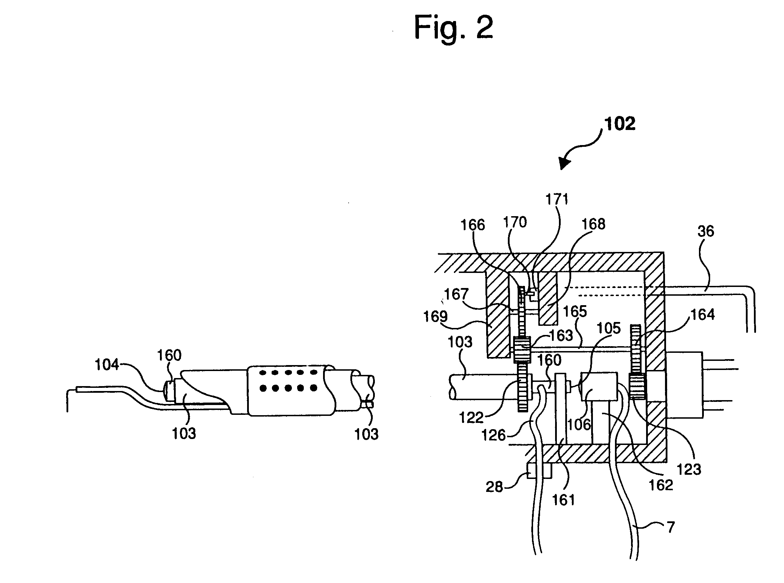

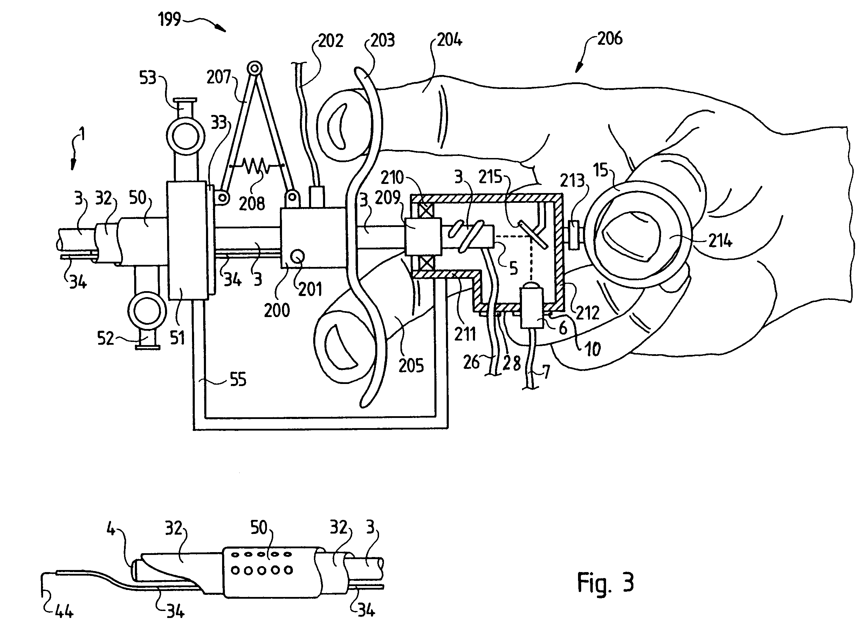

In der Zeichnung ist die Erfindung beispielsweise und schematisch dargestellt. Die Figuren 1 und 2 zeigen in stark schematisierter Achsschnittdarstellung Ausführungsvarianten einer ersten Ausführungsform des erfingungsgemäßen Endoskopes, während Fig. 3 in Ansicht gemäß Fig. 1 eine konstruktiv stark vereinfachte zweite Ausführungsform zeigt.

Das in Figur 1 dargestellte Endoskop besteht im wesentlichen aus einem Schaftteil 1 und einem Endkörper 2, der am proximalen Ende des Schaftteiles 1 angeordnet ist.

Im Schaftteil 1 ist eine Optik in einem Optikrohr 3 vorgesehen, das an seinem distalen Ende ein Objektiv 4 aufweist und an seinem proximalen Ende 5 offen ist. Es wird dort von einer seitlich stehenden Videokamera 6 mit Anschlußkabel 7 über ein Umlenkprisma 8 betrachtet, welches mit der Kamera 6 über eine Stütze 9 verbunden ist.

Die Kamera 6 ist einsteckbar in einem Stutzen 10 in der Seitenwand eines Gehäuses 11 angeordnet. Das Gehäuse 11 ist drehbar in einer Nut 12 eines Lagerstückes 13 gelagert, welches distal vom proximalen Ende 5 auf dem Optikrohr 3 befestigt ist. Die Lagerachse, um die das Gehäuse 11 dreht, fällt mit der optischen Achse der im Optikrohr 3 vorgesehenen Optik zusammen. Die Kamera 6 kann also mit dem Gehäuse 11 unter Aufrechterhaltung des optischen Einblickes um das Optikrohr 3 gedreht werden.

Am proximalen Ende des Endkörpers 2 außerhalb des Gehäuses 11 ist eine Griffeinrichtung 14 vorgesehen, die einen proximalen Griffteil und einen distalen Griffteil aufweist. Der proximale Griffteil ist als Daumenring 15 ausgebildet, während der distale Griffteil aus einer Griffstange 16 besteht. Der Daumenring 15 ist bei 17 drehbar mit den proximalen Enden zweier Gleitstangen 18 verbunden, die parallel zur Lagerachse verlaufend an einem Drehstück 19 befestigt sind, mit dem die gesamte Griffeinrichtung 14 koaxial zur Lagerachse drehbar in einer Bohrung 20 des Gehäuses 11 gelagert ist. Die Griffstange 16 ist als Bewegungsteil der Griffeinrichtung 14 längsverschiebbar auf den Gleitstangen 18 gelagen und mit einer Feder 21 gegen deren proximales Ende abgestützt.

Die Griffeinrichtung 14 dient zur Drehsteuerung des im Schaftteil 1 vorgesehenen, im Ausführungsbeispiel mit dem Optikrohr 3 drehfest verbundenen Arbeitsinstrumentes. Sie muß also mit dem Arbeitsinstrument drehfest gekoppelt sein, was im Ausführungsbeispiel über das Optikrohr 3 erfolgt. Wie Figur 1 zeigt, endet jedoch das Optikrohr 3 an seinem proximalen Ende 5 im Abstand zur Griffeinrichtung 14. In diese Lücke greift die Kamera 6 mit dem Umlenkprisma 8.

Zur Drehübertragung sind auf dem proximalen Endbereich des Optikrohres 3 und auf dem im Inneren des Gehäuses 11 liegenden Ende des Drehstückes 19 der Griffeinrichtung 14 je ein gleich großes Zahnrad 22, 23 koaxial zur Lagerachse befestigt. Die beiden Zahnräder 22, 23 kämmen mit einer Zahnwalze 24, die mit ihrer Achse 25 am Gehäuse 11 parallel und außerhalb der Lagerachse drehbar gelagert ist. Auf diese Weise kann das Gehäuse 11 beliebig um das Optikrohr 3 und die Griffeinrichtung 14 gedreht werden, wobei zwischen letzteren über die Zahnwalze 24 eine drehstarre Kopplung aufrechterhalten bleibt, ohne daß die Drehkoppeleinrichtung mit dem Eingriff der Kamera in den Bereich der Hauptachse mit dem Umlenkprisma 8 kollidiert.

Das Optikrohr 3 weist in seinem Inneren einen flexiblen Lichtleiter 26 auf, der im Bereich des Objektives 4 in dessen Blickrichtung abstrahlend endet. Der Lichtleiter 26 ist bei 27 aus dem Optikrohr 3 herausgeführt, also an einer Stelle innerhalb des Gehäuses 11. Er verläuft von dort mehrfach um das Optikrohr 3 gewickelt und dann zu einer Durchführung 28 in der Seitenwand des Gehäuses 11, die vorteilhaft als Steckanschluß für ein weiterführendes Lichtleiterkabel ausgebildet ist.

Diese Art der Drehverbindung des Lichtleiters 26 zwischen dem Optikrohr 3 und dem Gehäuse 11 ermöglicht zwar einen großen Drehwinkel von z.b. 720°, erfordert aber dennoch eine Drehwinkelbegrenzung des Gehäuses 11 gegenüber dem Optikrohr 3.

Zur Drehwinkelbegrenzung über einen beliebigen Drehwinkelbereich ist ein Zahnrad 29 vorgesehen, das im Schneckeneingriff mit einer Schnecke 30 steht, welche parallel zur Achse 25 der Zahnwalze 24 im Gehäuse 11 drehfest angeordnet ist. An den Enden der Schnecke 30 sind Endanschläge 31 ausgebildet, gegen die das Zahnrad 29 bei seiner Schraubbewegung auf der Schnecke 30 in die Bewegung begrenzende Endanlage kommt. Das Zahnrad 29 kämmt mit der Zahnwalze 24 und wird bei dessen Drehbewegung auf der Schnecke 30 geschraubt. Gelangt es in Anschlag an einen der Endanschläge 31, so wird die weitere Verdrehung des Gehäuses 11 gegenüber dem Optikrohr 3 blockiert.

Im dargestellten Ausführungsbeispiel weist der Schaftteil 1 einen das Optikrohr 3 umgebenden Innenschaft 32 auf, der mit einem proximalen Endstück 33 am Optikrohr 3 und somit am Endkörper 2 befestigt ist. Innerhalb des Innenschaftes 32 ist eine Betätigungsstange 34 angeordnet, die das Endstück 33 längsverschiebbar durchsetzt und an einem Schiebestück 35 befestigt ist, welches auf dem Optikrohr 3 längsverschiebbar gelagert ist.

Das Schiebestück 35 wird zur Betätigung irgendwelcher im distalen Endbereich des Innenschaftes 32 vorgesehener Arbeitsinstrumente, wie zum Beispiel Zangen oder dergleichen, verwendet. Die Betätigung erfolgt vom Bewegungsteil der Griffeinrichtung 14, also im Ausführungsbeispiel von dessen Griffstange 16 her. In der dargestellten vorteilhaften Ausführung ist dazu eine Schiebestange 36 vorgesehen, die die Endwände des Gehäuses 11 in Bohrungen 37 gleitbar durchsetzt, also parallel zur Hauptachse längsverschiebbar im Gehäuse 11 gelagert ist. Quer zur Hauptachse abgewinkelte Endstücke 38 und 39 der Schiebestange 36 greifen mit Gabeln 38' bzw. 39' in Umfangsnuten 40 und 41 auf dem Schiebestück 35 bzw. der Griffstange 16. Bei Verdrehung des Gehäuses 11 um das Optikrohr 3 bleiben die Endstücke 38 und 39 der Schiebestange 36 mit ihren Gabeln in Eingriff mit den Umfangsnuten 40 und 41. Es läßt sich also stets eine Schiebeübertragung von der Griffstange 16 auf das Schiebestück 35 und somit auf die Betätigungsstange 34 erreichen.

Im dargestellten Ausführungsbeispiel ist als Arbeitsinstrument des Endoskopes eine hochfrequenzbeaufschlagte Schneidschlinge 44 am distalen Ende der Betätigungsstange 34 vorgesehen. Mit der Schneidschlinge 44 können unter Betrachtung durch das Okular 4 Gewebsschnitte angefertigt werden. Ein derart ausgerüstetes Endoskop wird in der Urologie als Resektor bezeichnet. Die Betätigungsslange 34 ist in diesem Falle als isolierter Leiter auszubilden.

Im Endkörper 2 des Endoskopes muß hierfür eine leitende Verbindung zu einem Ansschluß 45 am Gehäuse 11 geschaffen werden, der als Stecker für ein weiterführendes HF-Kabel ausgebildet ist.

Am proximalen Ende der Betätigungsstange 34, die an dem längsverschiebbaren Schiebestück 35 befestigt ist, ist mittels eines längenveränderlichen schraubenförmigen Leiters 46 eine leitende Verbindung zum inneren Ende 47 einer leitenden Federspirale 48 geschaffen, die innerhalb des Gehäuses 11, das Optikrohr 3 umgebend, in einer quer zur Lagerachse liegenden Ebene ausgebildet ist und die mit ihrem äußeren Ende 49 am Anschluß 45 fixiert ist. Die Federspirale 48 greift mit ihrem inneren Ende 47 in eine Bohrung 56 des auf dem Optikrohr 3 feststehend angeordneten Lagerstückes 13 und ist dort gegen Drehbewegungen fixiert. Die Federspirale 48 kann nach Art einer Unruhe, wie sie bei mechanischen Taschenuhren verwendet wird, größere Drehwinkel zwischen ihrem inneren Ende 47 und ihrem äußeren Ende 49 ausgleichen, also die Drehbewegungen des Gehäuses 11 gegenüber dem Optikrohr 3 zulassen.

Im dargestellten Ausführungsbeispiel weist der Schaftteil 1 einen sämtliche innere Teile 3, 32, 34 umgebenden Außenschaft 50 auf, der proximal an einem Ring 51 befestigt ist, welcher auf dem Endstuck 33 des Innenschaftes 32 gelagert ist.

In üblicher Ausbildung als sogenanntes Dauerspülinstrument sind Spülwasseranschlüsse 52 und 53 vorgesehen, mit denen ständig Spülwasser durch den Innenraum des Innenschaftes 32 dem Operationsgebiet zugeführt und durch distale Öffnungen 54 im Außenschaft 50 abgesaugt wird.

In vorteilhafter Ausbildung ist dabei der Außenschaft 50 mit seinem proximalen Ring 51 auf dem Endstück 33, als gegenüber dem Optikrohr 3 frei drehbar gelagert. Er kann somit gegenüber dem Körper des Patienten, im Falle eines urologischen Resektoskopes also in der Harnröhre des Patienten, drehfest liegen bleiben, wenn die gesamten inneren Teile des Schaftteiles 1, insbesondere also das Optikrohr 3, verdreht werden.

Bei dieser Ausbildung kann der Außenschaft 50 koaxial zur Lagerachse des Gehäuses 11 drehbar gelagert sein und mit diesem zum Beispiel über die dargestellte Verbindungsstrebe 55, die an seinem Ring 51 befestigt ist, drehfest gekoppelt sein. Das Gehäuse 11 bleibt dann bei Verdrehungen des Optikrohres 3 und der Schneidschlinge 44 vermittels der Koppelung an den drehfest im Körper des Patienten gelagerten Außenschaftes 50 drehfest gehalten. Die vom Gehäuse 11 vorzugsweise nach unten abgehenden Kabel, also das Kabel 7 der Kamera 6, und die an der Durchführung 28 bzw. dem Stecker 45 abgehenden Kabel für die Lichtleitung und die HF-Zuführung, bleiben dann in ortsfester Position und stören den Operationsbetrieb auch bei Verdrehung des Instrumentes nicht.

Figur 2 zeigt ein Endoskop, das weitgehend der Figur 1 entspricht. Unveränderte Teile sind mit denselben Bezugszeichen wie in Figur 1 versehen. Es wird im folgenden nur auf die Konstruktionsdetails eingegangen, die in einer Variante dargestellt sind und die mit neuen Bezugszeichen bezeichnet sind, die soweit möglich, den Bezugszeichen ähnlicher Teile gemäß Figur 1 entsprechen, und zwar um 100 erhöht.

Bei der Konstruktion der Figur 1 ist die Optik mit ihrem Okular 4 drehfest im Optikrohr 3 angeordnet. Das Optikrohr 3 ist drehfest mit dem Arbeitsinstrument 34, 35, 44 gekoppelt. Die Optik dreht also mit dem Arbeitsinstrument, während die Kamera 6 mit dem Gehäuse 11 still steht.

Bei der Konstruktion der Figur 2 ist dies anders gelöst. Das Optikrohr 103 umschließt hier eine gesondert ausgebildete Optik 160 mit Objektiv 104. Die Optik 160 ist drehbar im Optikrohr 103 gelagert und an ihrem distalen Ende 105 an einer Halterung 161 drehfest gehalten, welche mit dem Gehäuse 11 verbunden ist. Das Optikrohr 103 dreht also mit dem Arbeitsinstrument, während die Optik 160 mit dem Gehäuse 11 dreht. Sie steht daher drehfest zur Kamera 106, die ebenfalls in einer Variante dargestellt ist. Im Gegensatz zur Ausführungsform der Figur 1 ist sie in der Lagerachse des Gehäuses 11 angeordnet und mit einem Arm 162 mit dein Gehäuse 11 verbunden.

Diese Ausführung kann optische Vorteile haben, da die Optik 104 drehfest zur Kamera 106 steht. Sie hat ferner konstruktive Vorteile dadurch, daß bei drehfest stehender Optik 160 der in dieser angeordnete Lichtleiter 126 nicht verdreht werden muß. Er geht auf direktem Wege in fester Verlegung zur Durchführung 28 am Gehäuse 11. Ebenso ist das Kabel 7 der Kamera 6 auf einfachem Wege durch die Wand des Gehäuses 11 verlegt, da die Kamera 106 mit dem Gehäuse 11 dreht.

Zur Drehübertragung zwischen der Griffeinrichtung 14 und dem Arbeitsinstrument bzw. dem mit diesem drehenden Optikrohr 3 und zur Drehwinkelbegrenzung ist an Hand der Figur 1 eine Konstruktion mit einem Zahnradgetriebe beschrieben. Hierzu ist in der Konstruktion der Figur 2 eine Variante dargestellt.

Auf dem proximalen Ende des Optikrohres 103 sitzt wiederum ein Zahnrad 122, das über das Zahnradgetriebe mit einem Zahnrad 123 zu koppeln ist, das drehfest mit der Griffeinrichtung 14 verbunden ist, wie gemäß Figur 1 beschrieben.

Die Koppelung dieser beiden Zahräder 122, 123 erfolgt hier jedoch nicht über eine Zahnwalze, sondern über Zahnräder 163 und 164, die mit den Zahnrädern 122 und 123 kämmen und auf einer gemeinsamen Verbindungswelle 165 befestigt sind, welche, wie die Zahnwalze 24, gemäß Figur 1 im Gehäuse 11 gelagert ist.

Wie der Figur 2 entnehmbar, sind die Zahnräder 122 und 123 unterschiedlich groß. Entsprechend, jedoch mit umgekehrtem Größenverhältnis, sind auch die Zahnräder 163 und 164 unterschiedlich groß. Es ergibt sich also eine Getriebeübersetzung zwischen der Griffeinrichtung 14 und dem Optikrohr 103 bzw. dem Arbeitsinstrument. Im dargestellten Falle ist die Übersetzung so gewählt, daß bei einer bestimmten Winkeldrehung der Griffeinrichtung 14 das Optikrohr 103 nur um einen sehr viel kleineren Winkel gedreht wird. Es kann also das Arbeitsinstrument sehr feinfühlig bewegt werden.

Genausogut ist auch eine Übersetzung "ins Schnelle" möglich, also derart, daß bei einer geringen Winkeldrehung der Griffeinrichtung 14 eine sehr viel größere Winkeldrehung des Arbeitsinstrumentes erfolgt. Das Arbeitsinstrument kann daher beispielsweise auch wie ein Bohrer oder Fräser schnell rotierend gedreht werden.

Die Drehwinkelbegrenzung war bei der Konstruktion der Figur 1 mit einem Zahnrad 29 auf einer Schnecke 30 zwischen Endanschlägen 31 ausgeführt. Auch hierzu zeigt Figur 2 eine Variante.

Mit einem der auf der Verbindungswelle 165 befestigten Zahnräder, im Ausführungsbeispiel mit dem Zahnrad 163, kämmt ein Zahnrad 166, das mit einer Achse 167 zwischen Stützen 168 und 169 gelagert ist, welche mit der Wand des Gehäuses 11 verbunden sind. Die Stütze 169 dient im Ausführungsbeispiel gleichzeitig als Lagerung für das eine Ende der Verbindungswelle 165.

Das Zahnrad 166 trägt auf einer Seite einen Stift 170. An der Stütze 168 ist ein Endanschlag 171 vorgesehen. Das Zahnrad 166 kann also uni etwas weniger als 360° drehen und gelangt dann stets mit der einen oder anderen Seite des Endanschlages 171 in Anlage.

Dadurch wird die Drehbewegung der Verbindungswelle 165 bzw. die Drehbewegung des Gehäuses 11 um das Optikrohr 103 begrenzt. Wie Figur 2 zeigt, ist das Zahnrad 166 von wesentlich größerem Durchmesser als das Zahnrad 122 auf dem Optikrohr 103. Entsprechend dem gewählten Übersetzungsverhältnis kann das Optikrohr 103 in dem Gehäuse 11 von Anschlagbegrenzung bis Anschlagbegrenzung einen wesentlich größeren Winkel als 360° ausführen.

Fig. 3 zeigt eine grundsätzlich andere Ausführungsform des erfindungsgemäßen Endoskopes, welche die konstruktiven Schwierigkeiten bei der Dreh- und Schiebebetätigung vermeidet.

Wie Fig. 3 zeigt, entspricht der Schaftteil 1 identisch dem der Ausführungsform der Fig. 1. Seine Teile sind daher mit denselben Bezugszeichen versehen. Der Endkörper 199 ist jedoch anders ausgebildet.

Das Endstück 33 ist, wie in der Ausführungsform der Fig. 1, mit dem Optikrohr 3 verbunden, das in seiner Ausbildung und Länge einschließlich des proximalen Endes 5 dem der Fig. 1 entspricht. Auf dem Optikrohr 3 ist ein Schiebestück 200 längsverschiebbar gelagert, das dem Schiebestück 35 der Fig. 1 entspricht. In dem Schiebestück 200 ist mit einer Klemmschraube 201 die Betätigungsstange 34 der Schneidschlinge 44 befestigt und der elektrische Leiter zur Stromversorgung der Schneidschlinge 44 mit einem vom Schiebestück 200 abgehenden HF-Kabel 202 elektrisch verbunden.

An dein Schiebestück 200 ist als distales Griffteil eine Griffstange 203 befestigt, die, wie die Fig. 3 zeigt, vom Zeigefinger 204 und Mittelfinger bzw. Ringfinger 205 der Hand 206 des Operateurs ergriffen wird.

Zwischen dem Endstück 33 des Schaftteiles 1 und dem Schiebestück 200 ist eine Gelenkbrücke 207 üblicher Bauart angeordnet mit Zugfeder 208. Die Gelenkbrücke zieht in der Ausführung des dargestellten Endoskopes als aktiv schneidender Resektor das Schiebestück 200 und somit die Schneidschlinge 44 in distale Richtung, während durch Eingriff der Finger an der Griffstange 203 die Schneidschlinge 44 in proximale Richtung gezogen werden kann.

In der Nähe des proximalen Endes 5 des Optikrohres 3 ist auf diesem ein Tragring 209 befestigt, auf dem ein schematisch angedeutetes Drehlager 210 läuft, welches ein rohrförmiges distales Endstück 211 eines Gehäuses 212 trägt, das in seiner Funktion grundsätzlich dem Gehäuse 11 der Fig. 1 entspricht. Das Gehäuse 212 ist über das Drehlager 210 frei drehbar auf dem Optikrohr 3 gelagert.

Das Gehäuse 212 trägt an seinem proximalen Ende über ein Drehlager 213 den Daumenring 15, der vom Daumen 214 des Operateurs ergriffen wird.

Wiederum entsprechend der Ausführungsform der Fig. 1 ist im frei drehbar gelagerten Gehäuse 212 die Kamera 6 mit Kabel 7 einsteckbar in der Umfangswand des Gehäuses 212 befestigt. Auch der Lichtleiter 26 ist entsprechend der Ausführungsform der Fig. 1 aus dem Optikrohr 3 in der Nähe von dessen proximalem Ende 5 heraus und durch die Wand des Gehäuses 212 nach außen geführt. Anstelle des Umlenkprismas 8 der Ausführungsform der Fig. 1 ist ein am Gehäuse 212 befestigter 45°-Spiegel 215 vorgesehen.

Bei der Ausführungsform der Fig. 3 wird das frei drehbare Gehäuse 212, wie die Figur zeigt, von der Hand 206 des Operateurs umgriffen. Mit den Fingern 204 und 205 wird die Griffstange 203 zur Drehsteuerung des Arbeitsinstrumentes ergriffen. Die Drehsteuerung muß daher bei dieser Konstruktion nicht wie bei der der Fig. 1 mit erheblichem konstruktivem Aufwand durch das Gehäuse 212 hindurch und unter Umgehung des Bereiches, in dem der Strahlengang der Kamera 6 in die Drehachse eingreift, vorgesehen sein.

Bei der Ausführungsform der Fig. 3 kann ebenfalls anstelle der Griffstange 203 und/oder anstelle des Daumenringes 15 ein anderes Griffteil vorgesehen sein, beispielsweise eine Scheibe, an deren Rand die Finger angreifen.

Der Lichtleiter 26 kann anstelle, wie in Fig. 3 dargestellt, durch das Gehäuse 212 verlegt zu sein, seinen Kabelabgang an anderer Stelle des Instrumentes haben, beispielsweise im Bereich des Endstückes 33. Dann wird der Kabelabgang des Lichtleiters zwar ständig gedreht, jedoch liegt er ebenso wie das HF-Kabel 202 distal von der gesamten Griffeinrichtung, also distal von der Griffstange 203, so daß er die Hand des Operateurs bei Verdrehung nicht stört.

Auch bei der Ausführungsform der Fig. 3 kann im Falle, daß der Außenschaft 50 drehbar auf dem Innenschaft 32 gelagert ist, der Ring 51 über die Verbindungsstange 55 mit dem Gehäuse 212 drehfest gekuppelt sein, so daß bei Drehbetätigung des Instrumentes der Außenschaft 50 mit den Spülanschlüssen 52 und 53 sowie die Kabelabgänge für den Lichtleiter 26 und das Kamerakabel 7 drehfest liegen bleiben.

Da das Gehäuse 212 bei der Ausführungsform der Fig. 3 die Kamera 6 und den Umlenkspiegel 215 trägt, ist es mit dem Drehlager 210 derart gelagert, daß seine Drehachse mit der optischen Achse des optischen Systems im Optikrohr 3 übereinstimmt. Da das Optikrohr 3 etwas exzentrisch im Schaftteil 1 sitzt, fällt die Drehachse des Gehäuses 212 nicht mit der Schaftachse zusammen, sondern liegt leicht versetzt parallel zu dieser.

## Patentansprüche

1. Starres medizinisches Endoskop mit einem Schaftteil (1) und einem am proximalen Ende des Schaftteiles angeordneten Endkörper (2, 102, 199),
wobei der Schaftteil (1) wenigstens ein Arbeitsinstrument (44, 34) und eine mit einem distalen Objektiv (4, 104) versehene Optik (3, 160) aufweist,
wobei der Endkörper (2, 102, 199) wenigstens ein außerhalb der Schaftachse vom Endkörper abgehendes Kabel (49, 26, 7, 126) aufweist
und wobei der Endkörper (2, 102, 199) eine Griffeinrichtung aufweist, die zwei in bezug auf die Schaftachse beabstandet angeordnete Teile (15, 16, 203) aufweist,
wobei der distale Griffteil (16, 203) drehfest an das Arbeitsinstrument (44, 34) gekoppelt ist
und der proximale Griffteil (15) zur Betätigung durch den Daumen (214) des Operateurs ausgebildet ist,
**dadurch gekennzeichnet**,
daß ein Gehäuse (11, 212) vorgesehen ist, das mit seiner Außenwand wenigstens in dem longitudinalen Bereich der Schaftachse, in dem das Kabel (49, 26, 7, 126) vom Endkörper abgeht, die Außenwand des Endkörpers (2, 102, 199) bildet,
wobei das Gehäuse (11, 212) gegenüber dem Arbeitsinstrument (44, 34) frei drehbar um eine Lagerachse gelagert ist, die in der Schaftachse liegt oder parallel zu dieser angeordnet ist
und wobei der proximale Griffteil (15) proximal vom Gehäuse (11, 212) angeordnet ist.

2. Endoskop nach Anspruch 1, **dadurch gekennzeichnet**, daß der distale Griffteil (203) distal vom Gehäuse (212) angeordnet ist.

3. Endoskop nach Anspruch 1, **dadurch gekennzeichnet**, daß der distale Griffteil (16) proximal vom Gehäuse (11) angeordnet ist.

4. Endoskop nach Anspruch 1, **dadurch gekennzeichnet**, daß an dem Gehäuse (11, 212) mehrere Kabel (26, 7) in wenigstens angenähert derselben Winkelstellung abgehen.

5. Endoskop nach Anspruch 1, **dadurch gekennzeichnet**, daß am Gehäuse (11, 212) das Kabel (7) einer distal von dem proximalen Griffteil (15) angeordneten, das proximale Ende (5, 105) der Optik (3, 160) betrachtenden Kamera (6, 106) abgeht.

6. Endoskop nach Anspruch 5, **dadurch gekennzeichnet**, daß die Kamera (6, 106) am Gehäuse (11, 212) befestigt ist und daß die Optikachse in der Lagerachse des Gehäuses (11, 212) liegt.

7. Endoskop nach Anspruch 1, **dadurch gekennzeichnet**, daß die Optik (3) drehfest mit dem Arbeitsinstrument (44, 34) gekoppelt ist.

8. Endoskop nach Anspruch 3, **dadurch gekennzeichnet**, daß das Arbeitsinstrument (44, 34) und der distale Griffteil (16) getrennt am Gehäuse (11) gelagert sind und über ein Zahnradgetriebe (22, 23, 24; 122, 163, 164, 123) mit außerhalb der Optikachse am Gehäuse (11) gelagerter Verbindungswelle (25, 165) drehverbunden sind.

9. Endoskop nach Anspruch 8, **dadurch gekennzeichnet**, daß das Zahnradgetriebe (22, 23, 24; 122, 163, 164, 123) mit einem am Gehäuse (11) gelagerten Zahnrad (29, 166) kämmt, dessen Drehwinkel durch Endanschläge (31; 171) begrenzt ist.

10. Endoskop nach Anspruch 8, **dadurch gekennzeichnet**, daß zur Betätigungsübertragung von der Griffeinrichtung (14) zum Arbeitsinstrument (44, 34) eine parallel zur Lagerachse im Gehäuse (11) längsverschiebbare Schiebestange (36) vorgesehen ist, deren Enden (39, 38) außerhalb des Gehäuses (11) in Dreheingriff mit zur Lagerachse koaxialen Flächen (41, 40) stehen, welche einerseits an einem axial verschiebbar gelagerten Bewegungsteil (16) der Griffeinrichtung (14) und andererseits an einem axial verschiebbaren Schiebestück (35) des Arbeitsinstrumentes (44, 34) vorgesehen sind.

11. Endoskop nach Anspruch 1, wobei der proximale Griffteil als Daumenring (15) ausgebildet ist, **dadurch gekennzeichnet**, daß der Daumenring (15) in der Lagerachse des Gehäuses (11, 212) angeordnet ist.

12. Endoskop nach Anspruch 1, **dadurch gekennzeichnet**, daß das innere Ende des Kabels (49, 26) an eine gegenüber dem Arbeitsinstrument (44, 34) drehfeste Einrichtung (13, 3) über eine Drehverbindung angeschlossen ist.

13. Endoskop nach Anspruch 12 mit einem flexiblen Lichtleiter (26) in der Optik (3), **dadurch gekennzeichnet**, daß der Lichtleiter (26) innerhalb des Gehäuses (11) mehrfach um den proximalen Endbereich der Optik (3) gewickelt und von dort zu einer Durchführung (28) in der Seitenwand des Gehäuses (11) geführt ist, durch die er das Gehäuse durchsetzt, um von dort vom Gehäuse abzugehen.

14. Endoskop nach Anspruch 12 mit einer zum Arbeitsinstrument (44, 34) führenden HF-Leitung (46) **dadurch gekennzeichnet**, daß das proximale Ende der HF-Leitung (46) innerhalb des Gehäuses (11) an das innere Ende (47) einer federelastischen, elektrisch leitenden Spirale (48) angeschlossen ist, die in einer Ebene quer zur Lagerachse stehend mit ihrem inneren Ende (47) am Arbeitsinstrument (34) und mit ihrem äußeren Ende (49) an einer Durchführung (45) in der Seitenwand des Gehäuses (11) fixiert ist, von der ein weiterführendes HF-Kabel vom Gehäuse abgehen kann.

15. Endoskop nach Anspruch 6, **dadurch gekennzeichnet**, daß die Kamera (6) quer zur Lagerachse in der Seitenwand des Gehäuses (11, 212) angeordnet ist und mit einer Strahlumlenkeinrichtung (8, 215) in die Lagerachse greift.

16. Endoskop nach Anspruch 1 mit einem den Schaftteil (1) umgebenden, diesem gegenüber drehbar angeordneten Außenschaft (50), **dadurch gekennzeichnet**, daß der Außenschaft (50) drehfest mit dem Gehäuse (11, 212) gekoppelt ist.

## Claims

1. Rigid medical endoscope including a shaft portion (1) and a terminal body (2, 102, 199) disposed at the proximal end of the shaft portion, wherein the shaft portion (1) has at least one working instrument (44, 34) and an optical system (3, 160), which is provided with a distal objective (4, 104), wherein the terminal body (2, 102, 199) has at least one cable (49, 26, 7, 126) leaving the terminal body outside the shaft axis and wherein the terminal body (2, 102, 199) has a gripping device, which has two portions (15, 16, 203) spaced apart with respect to the shaft axis, the distal gripping portion (16, 203) being rotationally fixedly coupled to the working instrument (44, 34) and the proximal gripping portion (15) being constructed for actuation by the thumb (214) of the operator, characterised in that a housing (11, 212) is provided whose outer wail constitutes the outer wall of the terminal body (2, 102, 199), at least in the longitudinal region of the shaft axis, in which the cable (49, 26, 7, 126) leaves the terminal body, the housing (11, 212) being mounted to be freely rotatable with respect to the working instrument (44, 34) about a bearing axis, which lies in the shaft axis or is disposed parallel to it and the proximal gripping portion (15) being disposed proximally of the housing (11, 212).

2. Endoscope as claimed in Claim 1, characterised in that the distal gripping portion (203) is disposed distally of the housing (212).

3. Endoscope as claimed in Claim 1, characterised in that the distal gripping portion (16) is disposed proximally of the housing (11).

4. Endoscope as claimed in Claim 1, characterised in that a plurality of cables (26, 7) pass out of the housing (11, 212) at approximately the same angular position.

5. Endoscope as claimed in Claim 1, characterised in that the cable (7) of a camera (6, 106), which is arranged distally of the proximal gripping portion (15) and views the proximal end (5, 105) of the optical system (3, 160) passes out of the housing (11, 212).

6. Endoscope as claimed in Claim 5, characterised in that the camera (6, 106) is secured to the housing (11, 212) and that the optical axis is situated in the bearing axis of the housing (11, 212).

7. Endoscope as claimed in Claim 1, characterised in that the optical system (3) is rotationally fixedly coupled to the working instrument (44, 34).

8. Endoscope as claimed in Claim 3, characterised in that the working instrument (44, 34) and the distal gripping portion (16) are mounted separately on the housing (11) and are rotationally connected via a gear set (22, 23, 24; 122, 163, 164, 123) to a connecting shaft (25, 165) mounted on the housing (11) outside the optical axis.

9. Endoscope as claimed in Claim 8, chracterised in that the gear set (22, 23, 24; 122, 163, 164, 123) meshes with a toothed wheel (29, 166) which is mounted on the housing (11) and whose angle of rotation is limited by end abutments (31; 171).

10. Endoscope as claimed in Claim 8, characterised in that for the purpose of actuation transmission from the gripping device (14) to the working instrument (44, 34) there is provided a push rod (36) which is longitudinally slidable in the housing (11) parallel to the bearing axis and whose ends (39, 38) are in rotary engagement outside the housing (11) with surfaces (40, 41), which are coaxial with the bearing axis and which are provided, on the one hand, on an axially slidably mounted movable portion (16) of the gripping device (14) and, on the other hand, on an axially slidable slider member (36) on the working instrument (44, 34).

11. Endoscope as claimed in Claim 1, wherein the proximal gripping portion is constructed as a thumb ring (15), characterised in that the thumb ring (15) is disposed in the bearing axis of the housing (11, 212).

12. Endoscope as claimed in Claim 1, characterised in that the inner end of the cable (49, 26) is connected by a rotary connection to a device (13, 3) which is rotationally fixed with respect to the working instrument (44, 34).

13. Endoscope as claimed in Claim 12 with a flexible light guide (26) in the optical system (3), characterised in that the light guide (26) is wound a number of times about the proximal end region of the optical system (3) and passes from there to a bush (28) in the side wall of the housing (11), through which it passes through the housing, in order to pass out of the housing from that position.

14. Endoscope as claimed in Claim 12 with an HF line (46) leading to the working instrument (44, 34), characterised in that the proximal end of the HF line (46) is connected within the housing (11) to the inner end (47) of a resilient elastic, electrically conductive coil (48), which is fixed upright in a plane transverse to the bearing axis with its inner end (47) on the working instrument (34) and with its outer end (49) on a bush (45) in the side wall of the housing (11), from which a further HF cable can pass out from the housing.

15. Endoscope as claimed in Claim 6, characterised in that the camera (6) is arranged in the side wall of the housing (11, 212) transverse to the bearing axis and extends into the bearing axis with a beam deflecting device (8, 215).

16. Endoscope as claimed in Claim 1 including an outer shaft (50), which surrounds the shaft portion (1) and is rotatably disposed with respect to it, characterised in that the outer shaft (50) is rotationally fixedly connected to the housing (11, 212).

## Revendications

1. Endoscope médical rigide avec une partie formant gaine (2) et un corps d'extrémité (2, 102, 199) disposé à l'extrémité proximale de cette partie gaine,
la partie gaine (1) présentant au moins un instrument d'opération (44, 34) et une optique (3, 160) munie d'un objectif distal (4, 104),
le corps d'extrémité (2, 102, 199) présentant au moins un câble (49, 26, 7, 126) partant du corps d'extrémité hors de l'axe de la gaine, et
le corps d'extrémité (2, 102, 199) présentant un dispositif servant de poignée et comportant deux parties (15, 16, 203) espacées relativement à l'axe de la gaine,
la partie distale (16, 203) de la poignée étant couplée à l'instrument d'opération (44, 34) sans possibilité de rotation par rapport à celui-ci, et
la partie proximale (15) de la poignée étant conformée de façon à pouvoir être actionnée par le pouce (214) de l'opérateur,
**caractérisé en ce qu'il**
est équipé d'un boîtier (11,212) formant avec sa paroi extérieure, au moins dans la partie longitudinale de l'axe de la gaine dans laquelle le câble (49, 26, 7, 126) part du corps d'extrémité, la paroi extérieure du corps d'extrémité (2, 102, 199),
le boîtier (11, 212) étant logé de façon à pouvoir tourner librement par rapport à l'instrument d'opération (44, 34) autour d'un axe de palier se trouvant dans l'axe de la gaine ou étant parallèle à celui-ci, et
la partie proximale (15) de la poignée étant placée en position proximale par rapport au boîtier (11, 212).

2. Endoscope selon la revendication 1**, caractérisé en ce que** la partie distale (203) de la poignée est placée en position distale par rapport au boîtier (212).

3. Endoscope selon la revendication 1, **caractérisé en ce que** la partie distale (16) de la poignée est placée en position proximale par rapport au boîtier (11).

4. Endoscope selon la revendication 1, **caractérisé en ce que** plusieurs câbles (26, 7) partent du boîtier (11, 212) sous un angle au moins approximativement identique

5. Endoscope selon la revendication 1, **caractérisé en ce que** le câble (7) d'une caméra (6, 106) placée en position distale par rapport à la pallie proximale (15) de la poignée et visant l'extrémité proximale (5, 105) de l'optique (3, 160) part du boîtier (11, 212).

6. Endoscope selon la revendication 5, **caractérisé en ce que** la caméra (6, 106) est fixée au boîtier (11, 212) et en ce que l'axe optique se trouve dans l'axe de palier du boîtier (11, 212).

7. Endoscope selon la revendication 1, **caractérisé en ce que** l'optique (3) est couplée à l'instrument d'opération (44, 34) de façon à ne pas pouvoir tourner par rapport à celui-ci.

8. Endoscope selon la revendication 3, **caractérisé en ce que** l'instrument d'opération (44, 34) et la partie distale (16) de la poignée sont logés séparément au niveau du boîtier (11) et couplés en accouplement tournant par une transmission par engrenage (22, 23, 24; 122, 163, 164, 123) avec un arbre de transmission (25, 165) logé dans le boîtier (11) en dehors de l'axe de l'optique.

9. Endoscope selon la revendication 8, **caractérisé en ce que** la transmission par engrenage (22, 23, 24; 122, 163, 164, 123) entraîne une roue dentée (29, 166) logée dans le boîtier (11) et dont l'angle de rotation est limité par des butées (31, 171) de fin de course.

10. Endoscope selon la revendication 8, **caractérisé en ce que ,** pour assurer la transmission de l'action du dispositif de poignée (14) à l'instrument d'opération (44, 34), il est prévu une tige coulissante (36) déplaçable longitudinalement et parallèlement à l'axe de palier dans le boîtier (11) et dont les extrémités (39, 38) se trouvent, en dehors du boîtier (11), en emprise tournante avec des surfaces (41, 40) coaxiales par rapport à l'axe de palier et conformées d'une part dans une partie mobile (16) du dispositif de poignée (14) logée de façon à pouvoir coulisser axialement et d'autre part dans une partie coulissante (35) axiale de l'instrument d'opération (44, 34).

11. Endoscope selon la revendication 1 dont la partie poignée proximale est conformée en anneau de pouce (15), **caractérisé en ce que** l'anneau de pouce (15) est disposé dans l'axe de palier du boîtier (11, 212).

12. Endoscope selon la revendication 1, **caractérisé en ce que** l'extrémité intérieure du câble (49, 26) est reliée par l'intermédiaire d'un accouplement tournant à un dispositif (13, 3) ne pouvant pas tourner par rapport à l'instrument d'opération (44, 34).

13. Endoscope selon la revendication 12 muni d'un câble optique (26) flexible dans l'optique (3), **caractérisé en ce que**, à l'intérieur du boîtier (11), le câble optique (26) est enroulé en plusieurs spires autour de la zone d'extrémité proximale de l'optique (3) d'où il est conduit vers un passage (28) dans le boîtier (11) duquel il sort par ce passage.

14. Endoscope selon la revendication 12 muni d'un conducteur H.F. vers l'instrument d'opération (44, 34), **caractérisé en ce que**, à l'intérieur du boîtier (11), l'extrémité proximale du conducteur H.F. (46) est connecté à l'extrémité intérieure d'une spirale (48) élastiquement déformable et électriquement conductrice qui, placée dans un plan transversal par rapport à l'axe de palier, est fixée par son extrémité intérieure (47) à l'instrument d'opération (34) et par son extrémité extérieure (49) à un passage (45) dans la paroi latérale du boîtier (11), un câble H.F. partant de ce passage.

15. Endoscope selon la revendication 6, **caractérisé en ce que** la caméra (6) est disposée transversalement par rapport à l'axe de palier dans la paroi latérale du boîtier (11, 212) et vise dans l'axe de palier au moyen d'un dispositif de déflexion des rayons lumineux.

16. Endoscope selon la revendication 1 avec une gaine externe (50) entourant la partie gaine (1) et disposée de façon à pouvoir pivoter par rapport à cette dernière, **caractérisé en ce que** la gaine externe (50) est couplée au boîtier (11, 212) de façon à ne pas pouvoir tourner par rapport à celui-ci.
